**Europäisches Patentamt**

**European Patent Office**

**Office européen des brevets**

(19)

(11) Veröffentlichungsnummer: **0 297 238 B1**

(12) ## EUROPÄISCHE PATENTSCHRIFT

(45) Veröffentlichungstag der Patentschrift:
27.12.90

(51) Int. Cl.⁵: **C07C 21/067,** C07C 17/10

(21) Anmeldenummer: **88106620.3**

(22) Anmeldetag: **26.04.88**

(54) Verfahren zur Herstellung von Methallylchlorid.

(30) Priorität: **30.06.87 DE 3721472**

(43) Veröffentlichungstag der Anmeldung:
**04.01.89 Patentblatt 89/1**

(45) Bekanntmachung des Hinweises auf die Patenterteilung:
**27.12.90 Patentblatt 90/52**

(84) Benannte Vertragsstaaten:
**BE DE FR GB IT NL**

(56) Entgegenhaltungen:
**JP-B-53 024 926**

**INDUSTRIAL AND ENGENEERING CHEMISTRY,
Band 31, 1939, Easton; J. burgin et al.: "Halogenation of
hydrocarbons; chlorination of olefins containing an
unsaturated tertiary carbon atom", Seiten 1413-1419
ULLMANNS "Encyclopädie der technischen
Chemie", 3. Auflage, Band 8 "Gefrierschutzmittel bis
Iridium", 1957; URBAN & SCHWARZENBERG,
München-Berlin, Seiten 352-356**

(73) Patentinhaber: **HÜLS AKTIENGESELLSCHAFT, - RSP
Patente / PB 15 - Postfach 13 20, D-4370 Marl 1(DE)**

(72) Erfinder: **Jabrik, Julius, Helweg 17b,
D-4285 Raesfeld(DE)**
Erfinder: **Viehweger, Rolf, Heitbruch 14,
D-4270 Dorsten 11(DE)**
Erfinder: **Sticken, Gerhard, Dr., Am Gecksbach 32,
D-4270 Dorsten 11(DE)**

## Beschreibung

Methallylchlorid (3-Chlor-2-methyl-1-propen) wird technisch durch Umsetzen von Isobuten mit Chlor in der Gasphase hergestellt. Die Reaktion läuft in einem gekühlten Rohrreaktor bei Temperaturen um (möglichst unter) 100 °C und bei etwa Atmosphärendruck ab. Die erforderliche Reaktionszeit liegt im Bereich von 0,5 bis zu wenigen Sekunden. Zur Vermeidung der Weiterchlorierung des Methallylchlorids wird durchweg mit (geringem) Isobutenüberschuß gearbeitet. Die Einsatzstoffe werden dem Reaktor über eine Zweistoffdüse zugeführt. Die Verdüsung von flüssigem Isobuten als indirektes Kühlmittel hat sich nicht bewährt; sie führt zu verstärkter Bildung unerwünschter, höherchlorierter Produkte, was offenbar eine Folge der schlechten Vermischung der Reaktionspartner ist (Ullmanns Encyklopädie der technischen Chemie, 4. Auflage, Band 9, Seite 472 ff., Verlag Chemie, Weinheim 1975).

Von A. Striegler im Labormaßstab durchgeführte Versuche zur Herstellung von Methallylchlorid aus Isobuten und Chlor haben gezeigt, daß bei dieser Umsetzung Unregelmäßigkeiten auftreten, die sich durch einen plötzlichen, unerklärlichen Temperaturanstieg bemerkbar machen und eine deutliche Zunahme der unerwünschten, höherchlorierten Produkte zur Folge haben ("Erfahrungen bei der Chlorierung von Isobutylen", Chem. Techn. 9, 1957, Seite 523 ff.). Derartige Unregelmäßigkeiten wurden mit wechselnder Häufigkeit und Dauer auch im großtechnischen Maßstab beobachtet.

Sie lassen sich indes unterdrücken, wenn man dem Reaktionsgemisch bestimmte Mengen Sauerstoff zuspeist. Die Vorgehensweise wird in der DE-OS 34 02 446 ausführlich dargelegt. Obwohl die nach diesem Verfahren erforderlichen Sauerstoff-Mengen gering sind (0,001 bis 1 Vol.-%, bezogen auf die gasförmigen Einsatzstoffe), kann die Sauerstoff-Einspeisung zu Problemen führen, insbesondere wenn sie in Form von Luft erfolgt. Der zugegebene Sauerstoff verläßt nämlich das Reaktionssystem praktisch unumgesetzt und erscheint somit in der Produktaufarbeitung nach Auswaschen des Chlorwasserstoffs und Kondensation des verbleibenden Reaktionsproduktes als "Restgas-Strom". Je nach Aufarbeitungsverfahren ist dieser "Restgas-Strom" mit Chlorkohlenwasserstoffen und/oder nicht umgesetztem Isobuten beladen. Ersteres erfordert unter Berücksichtigung der allgemein verschärften Umweltschutz-Bestimmungen (in der Bundesrepublik Deutschland siehe z. B. Erste Allgemeine Verwaltungsvorschrift zum Bundesimmissionsschutzgesetz "Technische Anleitung zur Reinhaltung der Luft - TA Luft" in der Fassung vom 27.02.86) durchweg eine Abgasreinigung (Wäsche, Adsorptionsverfahren oder ähnliches), letzteres ergibt zusätzlich Probleme wegen der zu beachtenden Explosionsgrenzen Isobuten-Sauerstoff (bzw. Luft). Beides führt zu einer Erhöhung der Herstellkosten des Methallylchlorids.

Aufgabe des erfindungsgemäßen Verfahrens war es somit, die Umsetzung von Isobuten mit Chlor zu Methallylchlorid so zu führen, daß auch ohne die an sich äußerst wirksame Sauerstoffeinspeisung großtechnisch störungsfrei bei weitgehend konstanter Temperatur gearbeitet werden kann.

Diese Aufgabe wird erfindungsgemäß entsprechend den Angaben der Patentansprüche gelöst.

Wesentliches Merkmal der Erfindung ist der Einsatz eines Kreuzstromreaktors für die Herstellung von Methallylchlorid aus Isobuten und Chlor. Der Kreuzstromreaktor ist dadurch gekennzeichnet, daß zwei (hier gasförmige) Reaktionskomponenten in einem Strömungsrohr zur Reaktion gebracht werden, wobei die eine Komponente das Rohr von Anfang bis Ende in voller Menge durchströmt und die andere Komponente über die gesamte Rohrlänge verteilt anteilmäßig zudosiert wird, wobei die Summe der Teilströme die insgesamt erforderliche Menge der zweiten Komponente darstellt (vgl. E. Fitzer, W. Fritz, Technische Chemie - Eine Einführung in die chemische Reaktionstechnik, Springer Verlag, Berlin 1975, Seiten 75 bis 78 und Seiten 322 bis 324).

Bei der Herstellung von Methallylchlorid durch Umsetzen von Isobuten und Chlor in der Gasphase hat sich nun überraschend gezeigt, daß der Einsatz eines Kreuzstromreaktors eine stabile Reaktionsführung auch ohne Zuspeisung des bisher verwendeten Sauerstoffs gewährleistet.

Im allgemeinen läßt man gasförmiges Isobuten bei Temperaturen von 0 bis 80 °C und bei Atmosphären- oder leicht erhöhtem Druck (bis etwa 3 bar (abs.) durch ein mantelgekühltes Reaktionsrohr strömen und düst die erforderliche Menge Chlor (nahezu stöchiometrisch, in der Summe geringer molarer Unterschuß gegenüber Isobuten) über zwei oder mehr Stellen, vorzugsweise annähernd gleichmäßig verteilt auf die einzelnen Zugabestellen in diesen Isobutenstrom ein.

Es hat sich gezeigt, daß eine solche Fahrweise gegenüber der bisher praktizierten (gleichzeitige Zugabe von Isobuten und Chlor über eine Zweistoffdüse am Anfang des Rohrreaktors und gegebenenfalls Vorabzugabe des Sauerstoffs z.B. in den Chlorstrom) unerwartet auch zu einer leichten Erhöhung des Methallylchlorid-Gehaltes bei gleichzeitiger leichter Absenkung des Isocrotylchlorid-Gehaltes im Produktaustrag führt, was in der Folge der Produktaufarbeitung (siehe Ullmanns Encyklopädie der technischen Chemie, l. c.) eine spürbare Erhöhung der Methallylchlorid-Ausbeute bewirkt. Diese Ausbeutesteigerung ist besonders deutlich erkennbar, wenn die Zuführung der insgesamt erforderlichen Chlormenge über 3 und mehr, vorzugsweise 3 bis 5, Einleitstellen erfolgt. In jedem Falle sollten die Stellen der Chlorzugabe so gewählt werden, daß das (in Strömungsrichtung gesehen) davor zudosierte Chlor weitgehend oder nahezu vollständig umgesetzt worden ist.

Zur Erreichung hoher Methallylchlorid-Ausbeuten hat es sich überdies als zweckmäßig erwiesen, den Chlorstrom über so eng dimensionierte Einsteckrohre einzudüsen, daß man eine sehr hohe Einströmgeschwindigkeit erreicht, vorzugsweise von 150 bis ca. 240 m/s, also nahezu Schallgeschwindigkeit (bei 50 °C). Man kann das Chlor auch mit Überschallgeschwindigkeit eindüsen. Dies hat jedoch wegen des technischen Aufwandes weniger Interesse.

Die nachfolgenden Beispiele erläutern das erfindungsgemäße Verfahren.

Vergleichsbeispiel A

In einem mantelgekühlten Rohrreaktor von 48 m Länge werden Isobuten und Chlor im Molverhältnis 1,02:1 zu Methallylchlorid als Hauptreaktionsprodukt umgesetzt. Die Reaktionsteilnehmer werden dem Reaktor über eine am Rohranfang installierte Zweistoffdüse zugeführt. Der Druck liegt im Mittel bei 1,4 bar (abs.). Die Reaktion wird mit 1,20 Vol.-% Luft entsprechend 0,24 Vol.-% Sauerstoff "stabilisiert".

Nach einer Reaktionsstrecke von 4, 8, 12 und 16 m werden Temperaturen von 90, 87, 61 und 46 °C gemessen. Das kondensierte (Chlorwasserstofffreie) Reaktionsprodukt zeigt einen Methallylchlorid-Gehalt von 84,2 Gewichtsprozent und einen Isocrotylchlorid-Gehalt von 3,9 Gewichtsprozent.

Nach Unterbrechung der Luftzuspeisung steigt die Temperatur an den vorgenannten Meßstellen auf Werte von 340, 120, 72 und 55 °C an. Der Methallylchlorid-Gehalt im kondensierten Reaktionsprodukt sinkt auf 52,7 Gewichtsprozent ab. Es setzt starke Verrußung des Reaktors ein. Die Anlage muß abgestellt und gereinigt werden.

Beispiel 1

Die Herstellung von Methallylchlorid wird wie in Vergleichsbeispiel A beschrieben durchgeführt, jedoch erfolgt die Chloreinspeisung nunmehr zu drei gleichen Teilen nach 0 (also über die bisher benutzte Zweistoffdüse), 4 und 12 m Reaktionsstrecke. Sauerstoff wird nicht eingespeist. Die Chloreinströmgeschwindigkeit beträgt etwa 230 m/s.

Die Reaktion zeigt keinerlei Instabilitäten.

Nach 4, 8, 12, 16 und 24 m Reaktionsstrecke werden 44, 79, 42, 52 und 22 °C gemessen. Das kondensierte Reaktionsprodukt zeigt einen Methallylchlorid-Gehalt von 85,7 Gewichtsprozent und einen Isocrotylchlorid-Gehalt von 3,5 Gewichtsprozent.

Beispiel 2

Die Herstellung von Methallylchlorid gemäß Beispiel 1 wird so abgeändert, daß die erforderliche Chlormenge zu drei gleichen Teilen nach 4, 12 und 20 m Reaktionsstrecke eingebracht wird.

Unter diesen Umständen mißt man nach einer Reaktionsstrecke von 4, 8, 12, 16 und 24 m Temperaturen von 14, 68, 40, 52 und 52 °C. Der Methallylchlorid-Gehalt im kondensierten Reaktionsprodukt beträgt 86,5 Gewichtsprozent, der des Isocrotylchlorids 3,5 Gewichtsprozent. Der nach 12 und 20 m Reaktionsstrecke analysierte Restgehalt an Chlor (jeweils unmittelbar vor der Neuzugabe) zeigt Chlorumsätze von jeweils mehr als 90 % bezogen auf die bis dahin dosierte Chlormenge an.

Die Reaktion zeigt keinerlei Instabilitäten.

Beispiel 3

Die Herstellung von Methallylchlorid nach Beispiel 1 wird so verändert, daß die erforderliche Chlormenge zu vier gleichen Teilen nach 0, 4, 12 und 20 m Reaktionsstrecke eingedüst wird.

Auch diese Fahrweise zeigt keinerlei Instabilitäten im Reaktionsverlauf.

Nach einer Reaktionsstrecke von 4, 8, 12, 16 und 24 m werden Temperaturen von 48, 77, 42, 55 und 54 °C registriert. Das kondensierte Reaktionsprodukt zeigt 86,5 Gewichtsprozent Methallylchlorid und 3,4 Gewichtsprozent Isocrotylchlorid.

Beispiel 4

Die Herstellung von Methallylchlorid nach Beispiel 1 wird so verändert, daß die erforderliche Chlormenge zu nur zwei gleichen Teilen eingedüst wird, und zwar nach 4 und 20 m Reaktionsstrecke.

Es werden auch bei dieser Vorgehensweise keinerlei Instabilitäten im Reaktionsablauf beobachtet.

Die Temperaturmessung ergibt nach 8, 12 und 24 m Werte von 70, 58 und 58 °C. Im kondensierten Reaktionsprodukt findet man 85,4 Gewichtsprozent Methallylchlorid und 3,7 Gewichtsprozent Isocrotylchlorid.

**Patentansprüche**

1. Verfahren zur Herstellung von Methallylchlorid aus nahezu stöchiometrischen Mengen Isobuten und Chlor in der Gasphase, dadurch gekennzeichnet, daß man die Reaktion in einem Kreuzstromreaktor durchführt, wobei das Isobuten ein mantelgekühltes Reaktionsrohr durchströmt und Chlor an zwei oder mehr Stellen dieses Reaktionsrohres zugespeist wird.

2. Verfahren nach Anspruch 1, dadurch gekennzeichnet, daß die Verteilung des Chlors auf die einzelnen Zugabestellen annähernd gleichmäßig erfolgt.

3. Verfahren nach Anspruch 1 und 2, dadurch gekennzeichnet, daß die Zuführung der insgesamt erforderlichen Chlormenge über 3 bis 5 Einleitstellen erfolgt.

4. Verfahren nach einem der Ansprüche 1 bis 3, dadurch gekennzeichnet, daß die Chlorzuführstellen so gewählt werden, daß die jeweils eingespeiste Chlormenge bis zur nachfolgenden Zufuhrstelle weitgehend umgesetzt ist.

5. Verfahren nach einem der Ansprüche 1 bis 3, dadurch gekennzeichnet, daß die Chlorzufuhrstellen so gewählt werden, daß die jeweils eingespeiste Chlormenge bis zur nachfolgenden Zufuhrstelle nahezu vollständig umgesetzt ist.

6. Verfahren nach einem der Ansprüche 1 bis 5, dadurch gekennzeichnet, daß man das Chlor mit einer Geschwindigkeit von 150 bis 260 m/s eindüst.

**Claims**

1. A process for the preparation of methallyl chloride from substantially stoichiometric quantities of isobutene and chlorine in the gaseous phase, characterized in that the reaction is performed in a cross-flow reactor, the isobutene flowing through a jacket-cooled reaction tube and chlorine being supplied at two or more points on the reaction tube.

2. A process according to claim 1, characterized in that the chlorine is substantially uniformly distributed to the individual feed points.

3. A process according to claims 1 and 2, characterized in that the total quantity of chlorine required is supplied via 3 to 5 feed points.

4. A process according to one of claims 1 to 3, characterized in that the chlorine supply points are so selected that the particular quantity of chlorine supplied is substantially reacted before it reaches the following supply point.

5. A process according to one of claims 1 to 3, characterized in that the chlorine supply points are so selected that the particular quantity of chlorine supplied is practically completely reacted before it reaches the following supply point.

6. A process according to one of claims 1 to 5, characterized in that the chlorine is injected at a speed of 150 to 260 m/sec.

**Revendications**

1. Procédé d'obtention de chlorure de méthallyle à partir de quantités à peu près stoechiométiques d'isobutène et de chlore en phase gazeuse, caractérisé en ce que l'on exécute la réaction dans un réacteur à courants croisés dans lequel l'isobutène parcourt un tube de réaction refroidi par une jaquette et que le chlore est introduit en deux ou plus emplacements de ce tube de réaction.

2. Procédé selon la revendication 1, caractérisé en ce que la répartition du chlore sur les emplacements d'addition particuliers s'effectue d'une manière à peu près régulière.

3. Procédé selon les revendications 1 et 2, caractérisé en ce que l'amenée de la quantité de chlore nécessaire au total s'effectue par l'intermédiaire de 3 à 5 emplacements d'introduction.

4. Procédé selon l'une des revendications 1 à 3, caractérisé en ce que les emplacements d'amenée du chlore sont choisis, de sorte que la quantité de chlore introduite respectivement est mise en réaction dans une large mesure jusqu'à l'emplacement d'amenée subséquent.

5. Procédé selon l'une des revendications 1 à 3, caractérisé en ce que les emplacements d'amenée du chlore sont choisis, de sorte que la quantité de chlore introduite respectivement est mise en réaction à peu près complètement jusqu'à l'emplacement d'amenée subséquent.

6. Procédé selon l'une des revendications 1 à 5, caractérisé en ce que l'on injecte le chlore avec une vitesse de 150 à 260 m/s.